(19) Europäisches Patentamt
European Patent Office
Office européen des brevets

(11) Publication number: 0 225 172
A1

# (12) EUROPEAN PATENT APPLICATION

(21) Application number: 86309236.7

(22) Date of filing: 26.11.86

(51) Int. Cl.⁴: C 07 D 213/803
C 07 D 213/80

(30) Priority: 26.11.85 US 801725

(43) Date of publication of application:
10.06.87 Bulletin 87/24

(84) Designated Contracting States:
AT BE CH DE ES FR GB GR IT LI LU NL SE

(71) Applicant: A.H. ROBINS COMPANY, INCORPORATED
1407 Cummings Drive P.O. Box 26609
Richmond Virginia 23261-6609(US)

(72) Inventor: Lo, Young Sek
200 Tamarack Road
Richmond Virginia 23229(US)

(74) Representative: Sheard, Andrew Gregory et al,
Kilburn & Strode 30, John Street
London WC1N 2DD(GB)

(54) **Process for the preparation of 5-chloro and 5-bromo-2-hydroxynicotinicacids.**

(57) A process for the preparation of 5-halo-2-hydroxynicotinic acids having the formula

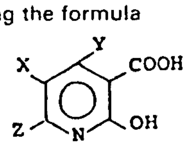

wherein X is chlorine or bromine; Y and Z are selected from hydrogen, loweralkyl, loweralkoxy, chlorine, bromine, iodine or fluorine, is described which comprises halogenating the corresponding 2-hydroxynicotinic acid with hypohalous acid in a concentrated halogen acid or with a alkali-metal hypohalite in aqueous or aqueous basic solution.

EP 0 225 172 A1

# PROCESS FOR THE PREPARATION OF 5-CHLORO AND 5-BROMO-2-HYDROXYNICOTINIC ACIDS

The present invention is concerned with a novel process for the preparation of certain 5-halo-2-hydroxynicotinic acids by halogenation of 2-hydroxynicotinic acids with hypohalous acid or alkali-metal salts thereof.

The halogenation of 2-hydroxynicotinic acids to produce 5-halo-2-hydroxynicotinic acids has not previously been disclosed in the literature.

The chlorination of 2-hydroxypyridine has recently been disclosed by A. R. Katritzky et al. in the J. ORG. CHEM. (1984) 49: pp 4784-4786 but not prior to the present invention. In that disclosure 5-chloro-2-pyridone which is equivalent to 5-chloro-2-hydroxypyridine was prepared by reacting 1 equivalent of 2-pyridone (the same as 2-hydroxypyridine) with 10 equivalents of sodium hypochlorite. In contrast, the 2-hydroxypyridines of the present invention are substituted in the three position by a carboxy radical.

The preparation of 5-chloro-2-hydroxynicotinic acid from 5-amino-2-hydroxynicotinic acid, hydrochloric acid and sodium nitrate is described in U. S. Patents 3,709,991 and 3,738,990.

H. M. Gilow in J. ORG. CHEM. (1974) vol. 39, 3481-3486 has reported on kinetics of bromination of some pyridinium ions such as 2,6-dimethylpyridinium to produce

3-bromo-2,6-dimethylpyridine using hypobromous acid in aqueous perchloric acid.

The solid products of the process of this invention i.e., compounds of Formula I, are useful as hypolipidemics as described in U. S. Patents 3,709,991 and 3,738,990. The compounds are also useful for the preparation of fused aromatic oxazepinones in copending application U. S. Serial No. 746,091, filed June 18, 1985, and U. S. Serial No. 753,325, filed August 9, 1985, which oxazepinones are useful as antihistaminics.

5-Halo-2-hydroxynicotinic acids prepared by the novel process of the invention have the formula:

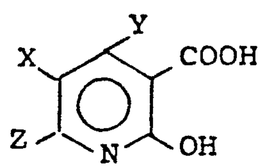

Formula I

wherein X is chlorine or bromine; Y and Z are selected from hydrogen, loweralkyl, loweralkoxy, chlorine, bromine, iodine or fluorine.

Aqueous solutions prepared in the novel process of the invention from which compounds of Formula I are isolated contain therein the equivalent of 5-halo-2-hydroxynicotinic acid compounds having the formula:

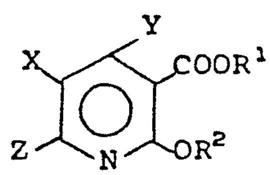

Formula II

wherein X, Y and Z are defined under Formula I and $R^1$ and $R^2$ are hydrogen or alkali-metals.

In the further definition of symbols in the formulas hereof and where they appear elsewhere throughout this specification and the claims, the terms have the following

significance.

The term "loweralkyl" as used herein, unless otherwise specified, includes straight and branched chain radicals of up to eight carbons inclusive and is exemplified by such groups as methyl, ethyl, propyl, isopropyl, butyl, sec.-butyl, tert.-butyl, amyl, isoamyl, hexyl, heptyl and octyl radicals and the like. The term "loweralkoxy" has the formula -O-loweralkyl.

The term "halo" as it applies to the general term 5-halo-2-hydroxynicotinic acids or compounds refers to chlorine or bromine. The "hypohalous" acids are hypochlorous and hypobromous acids. The "alkali-metal hypohalites" are sodium, potassium and lithium hypochlorite and hypobromite.

In the process of the invention, 5-halo-2-hydroxynicotinic acids or salts thereof (Formula II) are obtained in solution by halogenating 2-hydroxynicotinic acid compounds having the formula:

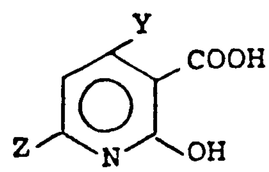

Formula III

using hypohalous acids or alkali-metal hypohalites.

When the hypohalous acids, i.e., hypochlorous and hypobromous acids, are used, the starting 2-hydroxynicotinic acid compounds are solubilized in a concentrated aqueous halogen acid, the halogen of which corresponds with the halogen of the hypohalite, and preferably the hypohalous acid is generated *in situ* by addition of concentrated hydrogen peroxide. Alternatively, hypohalous acids may be formed outside the reaction zone and bubbled into a solution of the 2-hydroxynicotinic acid compound in concentrated aqueous halogen acid. In any case, the halogenated product is also soluble in the solution due to protonation of the pyridine nitrogen and it may be precipitated by diluting the strong acid solution with water or by adjusting the pH upward with dilute aqueous base.

When alkali-metal hypohalites are used as halogenating agent, water is the solvent. Particularly high yields are obtained when alkali-metal hydroxides are used in combination with at least about 1 mole of alkali-metal base per mole of 2-hydroxynicotinic acid with optimum yields of chlorinated product being obtained at about 1.0 to 3.0 moles of alkali-metal base per mole of 2-hydroxynicotinic acid. Above about a 5:1 ratio the use of more base becomes uneconomical. The chlorinated product is soluble in the resulting solution and it may be precipitated by adding acid to neutralize the alkali-metal salt of the 5-hydroxy-2-hydroxynicotinic acid.

The following equations 1) and 2) set forth the chemistry involved in the chlorination reactions and the precipitation:

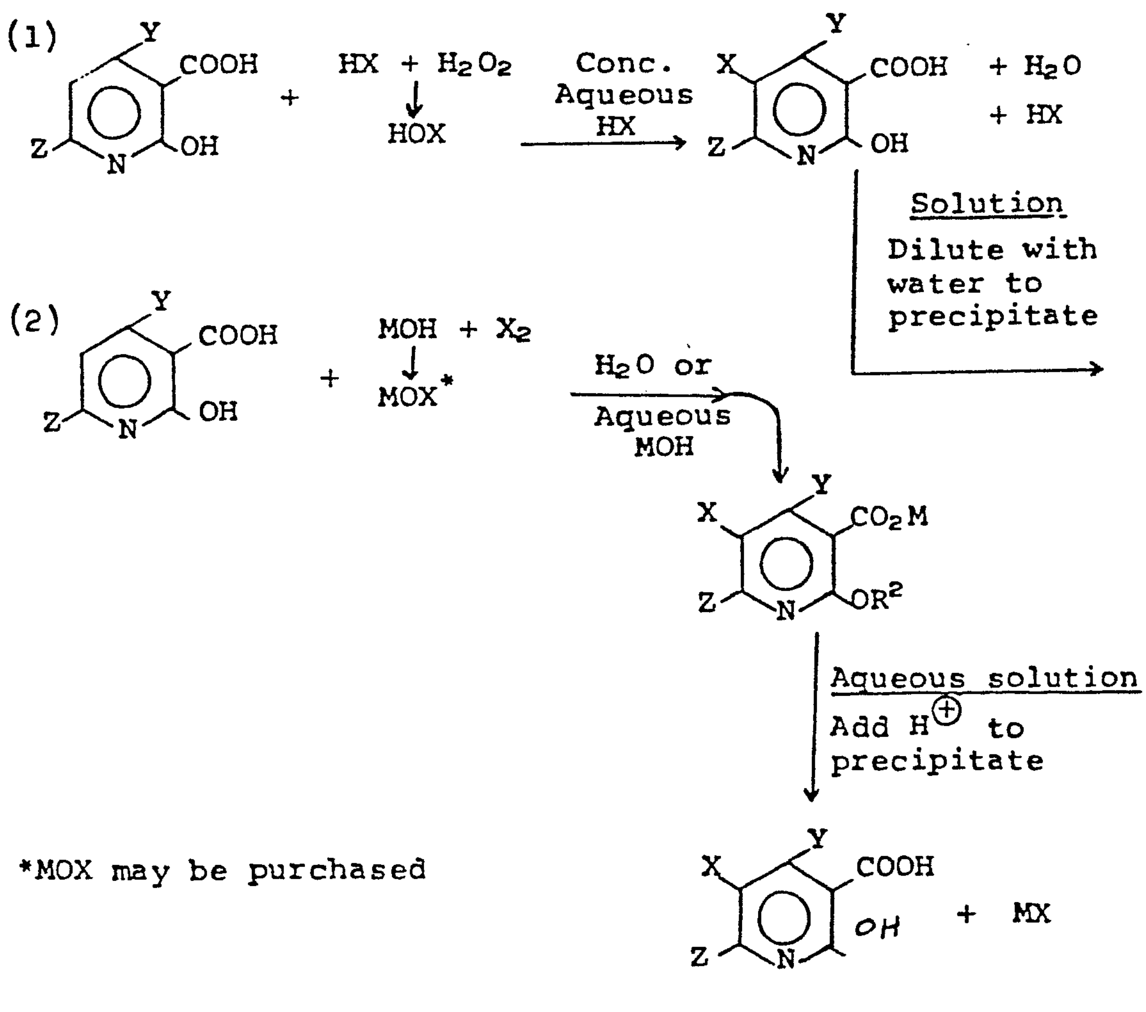


*MOX may be purchased

In equations 1) and 2), X is chlorine or bromine; $R^1$, $R^2$, Y, Z and M are as defined under Formulas I and II above. M is preferably sodium.

As shown above in equation 2), alkali-metal hypohalites may be prepared by reacting an alkali-metal hydroxide and halogen gas as was done in certain of the examples hereinbelow. The equation is

$$2\ MOH + X_2 \longrightarrow MOX + MX + H_2O$$

wherein M is alkali-metal and X is chlorine or bromine.

The novel process of the present invention in its most comprehensive aspect comprises the steps of:

Step 1, reacting a 2-hydroxynicotinic acid compound having the formula:

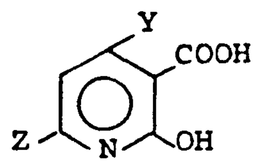

III

wherein Y and Z are selected from hydrogen, loweralkyl, loweralkoxy, chlorine, bromine, iodine or fluorine with one of the following hypohalous acids or hypohalite combinations a), b), or c):

alternative a) HOX + conc. aqueous HX,
b) aqueous MOX
or, c) aqueous MOX and MOH

wherein X is chlorine or bromine and M is alkali-metal to give a solution of a compound having the formula:

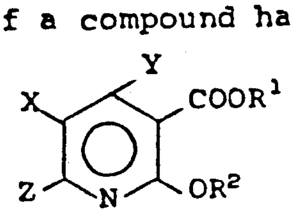

wherein X, Y and Z have the starting values of the reactants and $R^1$ and $R^2$ are hydrogen or alkali-metal

depending on whether alternative a), b), or c) was used.

Step 2, precipitating and separating a 5-halo-2-hydroxynicotinic acid compound having the formula:

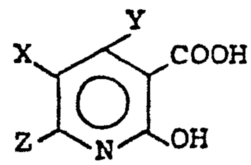

from the solution prepared in Step 1 by diluting the solution obtained in alternative a) with an amount of water or dilute base sufficient to precipitate the 5-halo-2-hydroxynicotinic acid compound and separating the precipitated product or adding a strong acid to a solution obtained in alternatives b) and c) in amount of said acid to precipitate the 5-halo-2-hydroxynicotinic acid compound and separating the precipitated product.

Step 1 is a novel method of preparing a solution of 5-chloro or bromo-2-hydroxynicotinic acids and alkali-metal salts thereof (Formula II) from which free acids may be isolated. Step 1 by itself is therefore an inventive entity or it may be joined with Step 2 to provide the more comprehensive process of Steps 1 and 2 combined.

In further reference to the process and process steps of the invention summarized above as they apply to the preparation of solutions of compounds of Formula II and separated solid compounds of Formula I, the following further description is applicable.

In Step 1, temperatures of about 0 to 50°C. are suitable, preferably about 0-25°C. for carrying out the chlorination. When an alkali-metal hypohalite is the halogenating agent, an improved yield of the 5-halo-2-hydroxynicotinic acid compound is obtained when the reaction solution contains alkali-metal base along with the alkali-metal hypohalite (alternative c) in an amount of about at least 1 mole of base per mole of starting 2-hydroxynicotinic acid compound and, as stated above, optimum

yields are obtained when the ratio is about 1.0 to 3.0, which ratio range is preferred. A further preferred procedure in carrying out Step 1, when alkali-metal hypohalite is used, is to add the hypohalite in two steps allowing a period of time of about 8-48 hr to lapse between additions. Sodium, potassium or lithium hypohalites may be used, sodium hypohalites being preferred.

In Step 2, alternative a), dilution with water alone is sufficient to precipitate the free acid compound, but weak alkaline solutions may be used as an obvious alternative. In alternatives b) and c), the amount of strong acid should be limited to that which will give maximum precipitation and should not be so much as to cause dissolution of the free acid due to protonation of the pyridine nitrogen. The excess alkali-metal hypohalites may be decomposed by adding a reducing agent such as sodium bisulfite or it may be decomposed by acid prior to precipitation of the 5-halo-2-hydroxynicotinic acid compound. The solids in all cases may be separated by filtration or centrifugation.

Examples 1 to 6 illustrate preparation of compounds encompassed by Formula I and solutions of compounds of Formula II. The scope of the invention is not limited by the examples, however.

Example 1

5-Bromo-2-hydroxy-3-pyridinecarboxylic acid.

To a solution of 10 g (0.07 mole) of 2-hydroxynicotinic acid in 16.8 g of 50% sodium hydroxide (0.21 mole) diluted with 25 ml of water was added 200 ml of sodium hypobromite solution prepared by adding 13.6 g (0.17 mole) of bromine to a solution of 20.16 g of 50% sodium hydroxide (0.25 mole) in 125 ml of water at 0°C. diluted to 400 ml. After 24 hrs of stirring at room temperature, another 100 ml portion of the above sodium hypobromite solution was added and the reaction solution was stirred for another 24 hr. The reaction solution was cooled in an ice bath and acidified carefully with 12 N hydrochloric acid to give a precipitate which was collected by filtration. Recrystallization from isopropyl alcohol gave 9.7 g (63.5%) of product. A sample was further recrystallized from 95% ethanol, m.p. 245°C.

Analysis: Calculated for $C_6H_4NO_3Br$: C,33.06; H,1.85; N,6.42
Found : C,32.98; H,1.83; N,6.44

Example 2

5-Chloro-2-hydroxy-3-pyridinecarboxylic acid.

2-Hydroxynicotinic acid was chlorinated in aqueous solution by using two additions in sequence (time interval between) of freshly prepared sodium hypochlorite solution resulting from reaction of chlorine and excess sodium hydroxide. The overall molar ratio of 2-hydroxynicotinic acid:sodium hypochlorite:excess sodium hydroxide in the chlorinating reaction was about 1:1.6:2.25 considering the totals in both additions. The detailed procedure used was as follows:

Sodium hydroxide, 336 g of 50% solution (4.2 mole) in 1 kg of ice was treated with bubbling chlorine gas while stirring until 78 g (1.1 mole) of chlorine was taken into the solution. To the resulting cold, basic sodium hypochlorite solution was added 142 g (1.0 mole) of 2-hydroxynicotinic acid in one portion. Within 1/2 hr the temperature had risen to 35°C. and dissolution had

occurred. The mixture was stirred overnight after which time $^{13}$CNMR analysis indicated the ratio of starting material to desired product (as the disodium salt) was about 30:70%. Another solution of basic sodium hypochlorite was prepared by the above procedure by absorbing 35 g (0.5 mole) of chlorine gas in a mixture of 100 g of 50% solution (1.25 mole) of sodium hydroxide and 278 g of ice. This hypochlorite solution was added to the main reaction mixture and stirring was continued overnight. $^{13}$CNMR analysis indicated the starting 2-hydroxynicotinic acid had all reacted and the desired 5-chloro-2-hydroxynicotinic acid was present as the disodium salt. Sodium bisulfite, 14 g was added and stirring was continued for 1.5 hr. The reaction mixture was gradually added to a cooled, stirred solution of 337 ml of concentrated (37%) hydrochloric acid. The maximum temperature of the acidified mixture was 25°C. Sodium hydroxide pellets (about 24 g) were slowly added so as to bring the mixture to pH slightly below pH=7. The yield of title compound on separation and drying was 146 g (84%).

The $^{1}$HNMR spectrum was obtained in DMSO-$d_6$ as follows:

| Chemical Shifts | Assignments |
|---|---|
| 13.20 ppm (broad singlet) | 2 protons |
| 8.20 ppm (multiplet) | 3 protons |

The $^{13}$CNMR spectrum was obtained in DMSO-$d_6$:

| Chemical Shifts | Assignments |
|---|---|
| 113.86 ppm | the beta-carbon bearing the carboxyl group |
| 117.76 ppm | the meta-carbon bearing the chlorine atom |
| 140.01 ppm | the unsubstituted carbon para to the pyridine nitrogen |
| 145.35 ppm | the unsubstituted carbon ortho to the pyridine nitrogen |
| 163.41 ppm<br>164.12 ppm | the carboxyl carbon and the ortho carbon bearing the hydroxyl group |

## Example 3

5-Chloro-2-hydroxy-3-pyridinecarboxylic acid.

2-Hydroxynicotinic acid was chlorinated in aqueous solution by using 2 additions in sequence (time interval between) of freshly prepared aqueous sodium hypochlorite solution resulting from reaction of chlorine and excess sodium hydroxide. The overall molar ratio of hydroxynicotinic acid: sodium hypochlorite: excess hydroxide in the chlorinating reaction was about 1:1.5:2.4 considering the totals in both additions. The detailed procedure used was as follows:

Sodium hydroxide, 10.05 kg of 50% solution (125.6 mole) in 30 kg of ice was treated with bubbling chlorine gas while stirring until 2.22 kg (31.3 mole) of chlorine was taken into the solution. To the resulting basic sodium hypochlorite solution was added 4.26 kg (30 mole) of 2-hydroxynicotinic acid. External cooling with tap-water was used to keep the temperature between 20-35°C. Stirring was continued over the weekend (72 hr) after which time $^{13}$CNMR analysis showed that about 70% of the 2-hydroxynicotinic acid was chlorinated. Another solution of basic sodium hypochlorite solution was prepared by the above procedure by absorbing 1.05 kg (14.8 mole) of chlorine gas in a mixture of 3.0 kg of 50% solution (37.5 mole) of sodium hydroxide and 8.3 kg of ice. This hypochlorite solution was added to the main reaction mixture and stirring was continued for about 12 hr, after which time $^{13}$CNMR indicated no 2-hydroxynicotinic acid remained. A solution of 104 g (1.0 mole) of sodium bisulfite in 300 ml of water was added to destroy the remaining sodium hypochlorite. The reaction mixture was added gradually to 9.9 liters (119 mole) of concentrated hydrochloric acid containing 2 liters of isopropyl alcohol (to control foam), keeping temperature between 13-25°C. The mixture was cooled and the precipitate was collected by filtration, washing with a small amount of water followed by 2 liters of acetone. The precipitate was dried to give 4.44 kg (85%) of the title compound, m.p. starts at 245°C. and decomposes at 250°C.

## Example 4

5-Chloro-2-hydroxy-3-pyridinecarboxylic acid.

A mixture of 7 g (0.05 mole) of 2-hydroxynicotinic acid in a 5% solution of sodium hypochlorite, 140 ml (0.094 mole), was stirred overnight at room temperature. Concentrated hydrochloric acid was added until the mixture became acidic. Precipitated solid was separated by filtration, rinsed 3 times with water, and air dried for 72 hr to give 4.42 g of solid. The solid was triturated 3 times with 6N hydrochloric acid and 3 times with water. The solid was dried under vacuum to give 2 g of product estimated by MS-CI to contain about 20% of starting 2-hydroxy-3-pyridinecarboxylic acid.

## Example 5

5-Chloro-2-hydroxy-3-pyridinecarboxylic acid.

2-Hydroxynicotinic acid was chlorinated with hypochlorous acid derived from mixing concentrated hydrochloric acid and hydrogen peroxide by the following procedure:

A solution of 14 g (0.10 mole) of 2-hydroxynicotinic acid in 70 ml of 37% hydrochloric acid was added dropwise to 16 ml of a 30% aqueous solution of hydrogen peroxide (0.15 mole), keeping the temperature of the reaction mixture between 23-45°C. (about 2/3 of the acid solution had been added in about 9 min and the remainder was added at a slower rate, total reaction time allowed being about 2 hr). The solid which precipitated was separated from the mother liquor by filtration, rinsed with water, and dried under vacuum to give the first crop, 24.8 g solid (37.7%). To the mother liquor was added 16 ml of 30% hydrogen peroxide (0.8 mole) and the mixture was stirred overnight. The precipitate which formed in the mother liquor was separated by filtration and rinsed and dried under vacuum to give a second crop of 1.38 g solid (8.0%) The total yield of title compound was 26.2 g for an overall yield of 45.7% of title compound.

Example 6 a-i

When in the procedure of Example 4, the following are substituted for 2-hydroxynicotinic acid:

4-chloro-2-hydroxynicotinic acid,
6-chloro-2-hydroxynicotinic acid,
4,6-dichloro-2-hydroxynicotinic acid,
4,6-dibromo-2-hydroxynicotinic acid,
4-methyl-2-hydroxynicotinic acid,
6-methyl-2-hydroxynicotinic acid,
4-fluoro-2-hydroxynicotinic acid,
4-iodo-2-hydroxynicotinic acid,
4-methoxy-2-hydroxynicotinic acid,

there are obtained:

a) 4,5-dichloro-2-hydroxy-3-pyridinecarboxylic acid,
b) 5,6-dichloro-2-hydroxy-3-pyridinecarboxylic acid,
c) 4,5,6-trichloro-2-hydroxy-3-pyridinecarboxylic acid,
d) 5-chloro-4,6-dibromo-2-hydroxy-3-pyridinecarboxylic acid,
e) 5-chloro-4-methyl-2-hydroxy-3-pyridinecarboxylic acid,
f) 5-chloro-6-methyl-2-hydroxy-3-pyridinecarboxylic acid,
g) 5-chloro-4-fluoro-2-hydroxy-3-pyridinecarboxylic acid,
h) 5-chloro-4-iodo-2-hydroxy-3-pyridinecarboxylic acid,

and, i) 5-chloro-4-methoxy-2-hydroxy-3-pyridinecarboxylic acid.

Various modifications and equivalents will be apparent to one skilled in the art and may be made in the process without departing from the spirit and scope thereof and it is therefore understood that the invention is to be limited only by the scope of the appended claims.

CLAIMS

1. A process for the preparation of a 5-halo-2-hydroxynicotinic acid compound having the formula:

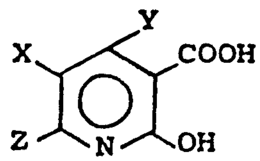

wherein X is chlorine or bromine, and Y and Z are selected from hydrogen, loweralkyl, loweralkoxy, chlorine, bromine or fluorine, which comprises the steps of

Step 1, reacting a 2-hydroxynicotinic acid compound having the formula:

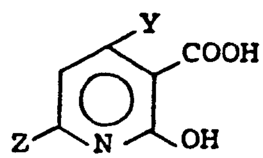

wherein Y and Z are selected from hydrogen, loweralkyl, loweralkoxy, chlorine, bromine or fluorine with one of the following hypohalous acid or hypohalite combinations a), b), or c):

alternative a) HOX plus conc. aqueous HX,

b) Aqueous MOX,

c) Aqueous MOX and MOH

wherein X is chlorine or bromine and M is alkali-metal to give a solution of a compound having the formula:

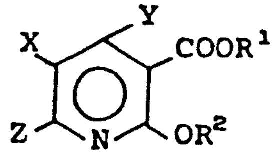

wherein X, Y and Z have the starting values of the reactants and $R^1$ and $R^2$ are hydrogen or alkali-metal depending on whether a), b), or c) was used; and optionally

Step 2, diluting the solution obtained in Step 1, alternative a), with an amount of water dilute base sufficient to precipitate the 5-halo-2-hydroxynicotinic acid compound and separating the precipitated product or adding a strong acid to a solution obtained in Step 1, alternative b) or c), in an amount of said acid to precipitate the 5-halo-2-hydroxynicotinic acid compound and separating the precipitated product.

2. A process as claimed in claim 1 wherein the hypohalous composition used in an alkali metal hypohalite.

3. A process as claimed in claim 1 wherein the hypohalous composition is sodium hypochlorite.

4. A process as claimed in claim 1, 2 or 3 wherein the hypohalous composition used is a mixture of alkali-metal hypohalite and an amount of alkali-metal base corresponding to about 1.0 to 3.0 moles of said base per mole of said starting 2-hydroxynicotinic acid compound.

5. A process as claimed in any one of claims 1 to 4, wherein the compound prepared is 5-bromo-2-hydroxy-3-pyridinecarboxylic acid.

6. A process as claimed in any one of claims 1 to 4, wherein the compound prepared is 5-chloro-2-hydroxy-3-pyridinecarboxylic acid.

7. A process for the preparation of a 5-halo-2-hydroxynicotinic acid compound having the formula

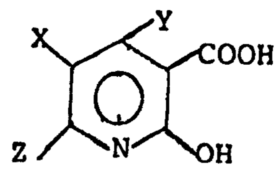

wherein X is chlorine or bromine; and Y and Z are selected from hydrogen, loweralkyl, loweralkoxy, chlorine, bromine, iodine or fluorine, which comprises reacting in a concentrated aqueous solution of hydrogen chloride or hydrogen bromide, a 2-hydroxynicotinic acid compound having the formula

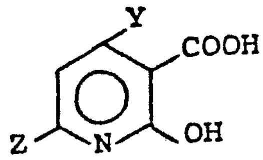

wherein Y and Z are as defined above with hypochlorous acid or hypobromous acid and thereafter adding sufficient water to precipitate the said 5-halo-2-hydroxynicotinic acid compound.

8. A process for the preparation of 5-halo-2-hydroxynicotinic acid compound having the formula

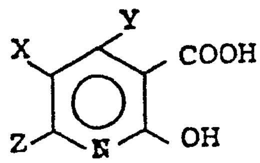

wherein X is chlorine or bromine, Y and Z are selected from hydrogen, loweralkyl, loweralkoxy, chlorine, bromine, iodine or fluorine, which comprises adding a solution of a 2-hydroxynicotinic acid compound having the formula

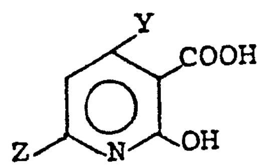

in concentrated hydrochloric or hydrobromic acid to an aqueous solution of concentrated hydrogen peroxide and thereafter separating said 5-halo-2-hydroxynicotinic acid from the solution and collecting it.

9. A process for the preparation of a 5-halo-2-hydroxynicotinic acid compound having the formula

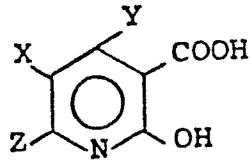

wherein X is chlorine or bromine; and Y and Z are selected from hydrogen, loweralkyl, loweralkoxy, chlorine, bromine, iodine or fluorine, which comprises reacting in an aqueous solution a 2-hydroxynicotinic acid compound having the formula

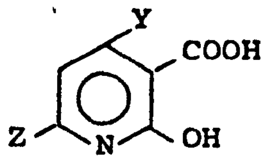

wherein Y and Z are as defined above with a solution containing an alkali-metal hypohalite of the formula

MOX

wherein X is chlorine or bromine and M is sodium or potassium, said solution containing 1.0 to 3.0 moles of a MOH base wherein M is sodium or potassium for a time sufficient to effect halogenation of said 2-hydroxynicotinic acid compound and thereafter acidifying to a pH low enough to precipitate said 5-halo-2-hydroxynicotinic acid but not so as to dissolve it and collecting said precipitate.

10. A process for the preparation of an aqueous solution comprised of water and a 5-halo-2-hydroxynicotinic acid compound having the formula

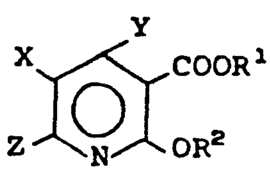

wherein X is chlorine or bromine; Y and Z are selected from hydrogen, loweralkyl, loweralkoxy, chlorine, bromine, iodine or fluorine, and $R^1$ and $R^2$ are hydrogen or alkali-metal, which comprises reacting a 2-hydroxynicotinic acid compound having the formula

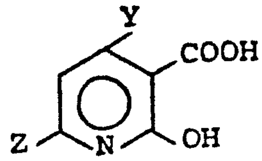

wherein Y and Z are selected from hydrogen, loweralkyl, loweralkoxy, chlorine, bromine, iodine or fluorine with one of the following hypohalous acid or hypohalite combinations a), b) or c):

alternative a) HOX plus conc. aqueous HX,

b) Aqueous MOX,

c) Aqueous MOX + MOH

wherein X is chlorine or bromine and M is alkali-metal.

European Patent Office

# EUROPEAN SEARCH REPORT

0225172

Application number

EP 86309236.7

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | GB - A - 1 357 559 (CIBA-GEIGY) * Claims 1,10,13; example 1 * -- | 1,2,3 | C 07 D 213/803 C 07 D 213/80 |
| A | US - A - 2 532 055 (BRUCE) * Claim 1 * -- | 1 | |
| D,A | US - A - 3 738 990 (BEAMAN) * Abstract; example 3 * ---- | 1 | |

TECHNICAL FIELDS SEARCHED (Int Cl 4)

C 07 D 213/00

The present search report has been drawn up for all claims

| Place of search | Date of completion of the search | Examiner |
|---|---|---|
| VIENNA | 27-02-1987 | HOCHHAUSER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document